# EUROPÄISCHE PATENTANMELDUNG

(11) **EP 0 997 471 A2**
(43) Veröffentlichungstag der Anmeldung: **03.05.2000**
(21) Anmeldenummer: 99250312.8
(22) Anmeldetag: 08.09.1999
(51) Int. Cl.: C07H 15/10, C07H 15/20

(54) **Verfahren zur Herstellung von Glycosiden**

(30) Priorität: 30.09.1998 SI 9800253
(71) Anmelder: H.F. & Ph.F. Reemtsma GmbH, 22605 Hamburg (DE)
(72) Erfinder: Kröger, Lars, 22457 Hamburg (DE); Thiem, Joachim, 22391 Hamburg (DE); Rudolph, Gert, Dr., 22587 Hamburg (DE); Pienemann, Thomas, 21224 Rosengarten (DE)
(74) Vertreter: UEXKÜLL & STOLBERG

(57) **Zusammenfassung**

Die Erfindung betrifft ein Verfahren zur Herstellung von Glycosiden aus einem O-geschützten Halogenid eines Einfach- oder Mehrfachzuckers, insbesondere α-Acetobromglucose, und einem Enol oder Phenol, insbesondere Vanillin oder Ethylvanillin, bei dem man die Herstellung in einem zweiphasigen Lösungsmittelgemisch in Gegenwart eines Phasentransferkatalysators durchführt. Mit dem erfindungsgemäßen Verfahren ist es unter Einsatz preiswerter und physiologisch unbedenklicher Reagentien möglich, Glycoside mit einer Ausbeute von 59 % zu synthetisieren.

## Beschreibung

Die Erfindung betrifft ein Verfahren zur Herstellung von Glycosiden aus einem O-geschützten Halogenid eines Einfach- oder Mehrfachzuckers und einem Alkohol, bei dem man die Reaktion in einem zweiphasigen Lösungsmittelgemisch in Gegenwart eines Phasentransferkatalysators durchführt.

Glycoside sind in der Natur weit verbreitete Verbindungen mit teilweise interessanten physiologischen Eigenschaften. Sie bestehen aus einem oder mehreren Zuckern und aus einem oder mehreren Aglykonen (d.h. Nichtzuckern), die über eine sogenannte glycosidische Etherbindung an ein Halbacetal-C-Atom gebunden sind. Bei höheren Temperaturen können sich Glycoside unter Abspaltung des Aglycons zersetzen. Diese Eigenschaft wird beispielsweise in US-A-4,804,002 ausgenutzt, um aus einem Ethylvanillinglycosid bei höheren Temperaturen die Vanillinkomponente zum Zweck der Aromatisierung freizusetzen.

In der gleichen Schrift wird ein Syntheseverfahren für das verwendete Ethylvanillyl-β-D-glucopyranosid beschrieben, das jedoch sehr aufwendig ist und nur zu schlechten Ausbeuten führt.

Auch unter Verwendung anderer, bereits bekannter Verfahren wie beispielsweise der Imidatmethode (R. Schmidt und W. Kinzy, *Adv. Carbohydr. Chem. Biochem.* **1994**, *50,* 121-123), der Umsetzung des Glucosepentaacetats unter Lewis-Säure-Katalyse (J. Dahmén et al., *Carbohydr. Res.* **1983**, *114*, 328-330), der Koenigs-Knorr-Methode (H. Paulsen, *Angew. Chem.* **1982**, *94*, 184-201) und der Fischer-Raske-Synthese (E. Fischer und K. Raske, *Ber. Dtsch. Chem. Ges.,* **1909**, *42,* 1465-1476) konnten gewünschte Glycoside wie beispielsweise Ethylvanillyl-β-D-glycopyranosid lediglich in schlechten Ausbeuten von unter 40 % hergestellt werden.

Ziel der vorliegenden Erfindung ist es somit, ein Verfahren zur Verfügung zu stellen, mit dem man Glycoside aus einem O-geschützten Einfach- oder Mehrfachzucker und einem Alkohol in guten Ausbeuten unter Einsatz preiswerter und physiologisch unbedenklicher Reagentien herstellen kann.

Erfindungsgemäß wird zur Lösung der Aufgabe das Verfahren nach Anspruch 1 vorgeschlagen. Vorteilhafte Ausgestaltungen sind in den Ansprüchen 2 bis 11 definiert. Das erfindungsgemäße Verfahren zeichnet sich dadurch aus, daß man
a) ein O-geschütztes Halogenid eines Einfach- oder Mehrfachzuckers (I) und einen ungesättigten Alkohol (II), der das Strukturelement 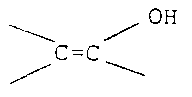 enthält,
b) in einem zweiphasigen Lösungsmittelgemisch und
c) in Gegenwart eines Phasentransferkatalysators zur Reaktion bringt
d) und gegebenenfalls das erhaltene geschützte Glycosid entschützt.

Im Rahmen der Erfindung hat es sich überraschenderweise gezeigt, daß durch Einsatz der Phasentransferkatalyse die Herstellung von Glycosiden mit einer bisher unbekannten Ausbeute von 59 % möglich ist. Das erfindungsgemäße Verfahren zeichnet sich durch eine breite Anwendbarkeit aus, da es für die Glycosidierung einer Vielzahl von ungesättigten Alkoholen eingesetzt werden kann. Ferner hat das erfindungsgemäße Verfahren den Vorteil, daß preisgünstige, gut verfügbare und nicht-toxische Edukte und Reagentien eingesetzt werden.

Unter einem O-geschützten Halogenid eines Einfach-oder Mehrfachzuckers wird im Sinne dieser Erfindung jedes α- oder β-Halogenid eines Einfach- oder Mehrfachzuckers verstanden, bei dem alle freien Hydroxylgruppen geschützt sind.

Unter Phasentransferkatalyse (vgl. z.B. R. Roy und F. Tropper, *J. Carbohydr. Chem.* **1985**, *4*, 2097-2102) wird im Sinne der Erfindung die Beschleunigung von Reaktionen in einem zweiphasigen Reaktionsgemisch verstanden.

Unter Phasentransferkatalysatoren werden im Sinne dieser Erfindung katalytisch wirksame Substanzen verstanden, die in einem zweiphasigen Reaktionsgemisch die Reaktion von in unterschiedlichen Phasen befindlichen Reaktanden an der Grenzfläche beschleunigen können.

Bevorzugt werden Halogenide von Einfach- oder Mehrfachzuckern eingesetzt, die O-acetyliert sind, d.h., bei denen Acetylgruppen als Schutzgruppen eingesetzt werden. Besonders bevorzugt wird für (I) α-Acetobromglucose eingesetzt.

Erfindungsgemäß können in dem Verfahren alle ungesättigten Alkohole (II) eingesetzt werden, die das Strukturelement 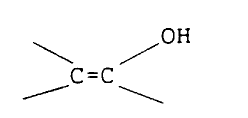 enthalten. Dabei ist es auch möglich, daß die Doppelbindung Bestandteil eines aromatischen oder nicht-aromatischen Ringsystems ist. Bevorzugt sind Alkohole, bei denen die Doppelbindung durch Konjugation mit anderen Doppelbindungen oder π-Systemen stabilisiert ist.

Gemäß einer bevorzugten Ausführungsform wird als ungesättigter Alkohol (II) ein Phenol der allgemeinen Formel 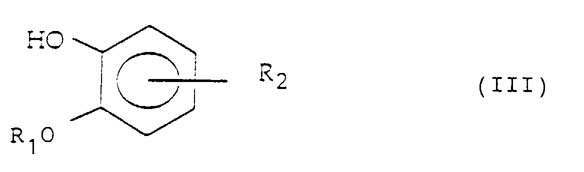 eingesetzt, wobei R₁ = Methyl, Ethyl, Propyl, i-Propyl, t-Butyl, Phenyl oder Benzyl und R₂ = -CHO, -CO-CH₃, -CO-C₂H₅, -CO-CH=CH₂, -CO-CH₂-CH=CH₂, -CO-CH=CH-CO₂H, -CO-CH=CH-CH₂OH, -CO-Phenyl oder -CO-Benzyl.

Überraschenderweise hat es sich gezeigt, daß die Phenole (III) aufgrund ihrer Basizität und Stabilität für die Glykosidierung von Einfach- oder Mehrfachzuckern durch Phasentransferkatalyse besonders geeignet sind.

In einer besonders bevorzugten Ausführungsform werden als Phenol (III) Vanillin oder Ethylvanillin eingesetzt. Erfindungsgemäß eingeschlossen ist weiterhin der Einsatz ähnlicher Verbindungen wie anderer Vanillinderivate, Eugenol oder Coniferylalkohol.

Gemäß einer weiteren bevorzugten Ausführungsform der Erfindung wird als ungesättigter Alkohol (II) ein ungesättigter Alkohol eingesetzt, der das Strukturelement 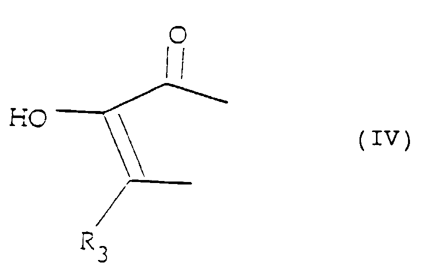 enthält, wobei R₃ = -H, Methyl, Ethyl, Propyl, i-Propyl, t-Butyl, Phenyl oder Benzyl.

Dabei kann die Doppelbindung Bestandteil eines Ringsystems sein. Bevorzugt ist die Doppelbindung Bestandteil eines gegebenenfalls teilhydrierten Furan-, Furanon-, Pyran- oder Pyronringes.

Überrraschenderweise hat es sich gezeigt, daß neben Phenolen (III) besonders ungesättigte Alkohole, die das Strukturelement (IV) enthalten und die zusätzlich durch eine α-Ketogruppe stabilisiert werden, aufgrund ihrer Basizität als Ausgangsmaterialien für das erfindungsgemäße Verfahrens besonders geeignet sind.

Gemäß einer besonders bevorzugten Ausführungsform wird als ungesättigter Alkohol (II), der das Strukturelement (IV) enthält, Maltol eingesetzt. Auch Maltol-ähnliche Verbindungen wie Ethylmaltol, andere Maltolderivate oder 4-Hydroxy-2,5-dimethyl-3(2H)-furanon (Furaneol®) können verwendet werden.

Die erfindungsgemäße Reaktion von Halogenid und Alkohol wird in einem zweiphasigen Lösungsmittelgemisch durchgeführt. Hierfür wird üblicherweise eine Mischung aus einem organischen und einem anorganischen Lösungsmittel eingesetzt. Besonders bevorzugt wird die Reaktion von Halogenid und Alkohol in einem Gemisch aus einer 1 N wäßrigen Natriumhydroxid-Lösung und Methylenchlorid durchgeführt.

Als anorganische Lösungsmittel kommen neben wäßriger Natriumhydroxidlösung auch wäßrige Lösungen von KOH, K₂CO₃, Na₂CO₃ und NH₃ in Frage, die bevorzugt als 1 N Lösungen eingesetzt werden.

Als organisches Lösungsmittel kommen neben Methylenchlorid Chloroform, Dichlorethan, Tetrachlorkohlenstoff, Diethylether, Tetrahydrofuran, Hexan, Petrolether sowie überkritisches CO₂ in Frage.

Die erfindungsgemäße Reaktion von Halogenid und Alkohol wird in Gegenwart eines Phasentransferkatalysators durchgeführt. Gemäß einer bevorzugten Ausführungsform handelt es sich bei diesem Phasentransferkatalysator um ein quartäres Ammoniumsalz und nach einer besonders bevorzugten Ausführungsform wird als Phasentransferkatalysator Tetrabutylammoniumbromid verwendet. Weitere geeignete Phasentransferkatalysatoren sind Tetrabutylammoniumhydrogensulfat, Tetrabutylammoniumchlorid, Tetrapropylammoniumbromid und Benzyl-tributylammoniumbromid.

Die Reaktion von Halogenid und Alkohol wird insbesonders bei einer Temperatur zwischen -10 °C und 100 °C durchgeführt. Bevorzugt wird die Reaktion bei einer Temperatur zwischen 5 °C und 40 °C durchgeführt, und besonders bevorzugt wird sie bei Raumtemperatur durchgeführt. Es ist vorteilhaft, die Reaktion unter ständigem Rühren oder Mischen durchzuführen.

Gemäß einer bevorzugten Ausführungsform der Erfindung werden die synthetisierten, O-geschützten Glycoside abschließend nach gängigen Methoden der Kohlenhydrat- und Schutzgruppenchemie entschützt.

Gemäß einer bevorzugten Ausführungsform sind die synthetisierten, geschützten Glycoside O-acetyliert. Diese O-acetylierten Glycoside werden erfindungsgemäß durch übliche Deacetylierungsreaktionen entschützt. Geeignet ist in diesem Zusammenhang vor allem eine Entschützung nach Zemplén (A. Thompson et al., *Meth. Carbohydr. Chem.* **1963**, *2*, 215-220).

Aus dem oben genannten ist deutlich geworden, daß das erfindungsgemäße Verfahren den Vorteil aufweist, daß Glycoside unter Verwendung preisgünstiger, gut verfügbarer und nicht-toxischer Edukte und Reagentien hergestellt werden können. Die dabei erzielbaren guten Ausbeuten sind in den Beispielen angegeben.

Im folgenden wird die Erfindung durch Beispiele erläutert.

### Beispiele

### Beispiel 1

41.1 g (100 mmol) 2,3,4,6-Tetra-O-acetyl-α-D-glucopyranosylbromid (α-Acetobromglucose), 32.3 g (100 mmol) Tetrabutylammoniumbromid und 33.3 g (200 mmol) 3-Ethoxy-4-hydroxybenzaldehyd (Ethylvanillin) werden in 350 ml Dichlormethan gelöst. Es werden dann 350 ml einer 1 molaren NaOH-Lösung zugegeben, und es wird eine Stunde bei Raumtemperatur heftig gerührt. Anschließend werden 3 l Ethylacetat zugegeben. Die organische Phase wird abgetrennt und nacheinander wird dreimal mit je 2 l einer 1 molaren NaOH-Lösung, dreimal mit je 2 l Wasser und einmal mit 2 l gesättigter Natriumchlorid-Lösung gewaschen. Die organische Phase wird über Magnesiumsulfat getrocknet, und das Lösungsmittel wird am Rotationsverdampfer entfernt. Zur Reinigung wird aus 95%-igem Ethanol umkristallisiert. Ausbeute: 29.34 g (59 %), Schmp.: 116 °C ¹H-NMR (CDCl₃, Bruker AMX-400): 9.84 (s, 1H, CHO), 7.39-7.34 (m, 2H, H-3', H-5'), 7.16 (d, 1H, H-6'), 5.34-5.23 (m, 2H, H-2, H-3), 5.16-5.11 (m, 1H, H-4), 5.10 (d, J=7.1, 1H, H-1), 4.23 (dd, 1H, H-6a), 4.15 (dd, 1H, H-6b), 4.07 (q, 2H, O-CH₂-CH₃), 3.83 (ddd, 1H, H-5), 2.03, 2.02, 2.01, 2.00 (je s, je 3H, OCOCH₃), 1.40 (t, 3H, O-CH₂-CH₃).

Zur Deacetylierung werden 39.6 g (79.76 mmol) Ethylvanillyltetra-O-acetyl-β-D-glucopyranosid in 400 ml abs. Methanol suspendiert und mit 40 ml einer 1%-igen NaOMe-Lösung versetzt. Nach 15 Minuten bildet sich ein voluminöser Niederschlag. Es wird mit Amberlite IR-120 (H⁺) neutralisiert und das Lösungsmittel entfernt. Die Umkristallisation aus 95%-igem Ethanol ergibt 24.3 g (93%) Ethylvanillyl-β-D-glucopyranosid. Schmp.: 202 °C. ¹H-NMR (d₄- MeOH, Bruker AMX-400): 9.74 (s, 1H, CHO), 7.45-7.39 (m, 2H, H-3', H-5'), 7.23 (d, 1H, H-6'), 5.00 (d, J=7.6, 1H, H-1), 4.09 (m, 2H, O-CH₂-CH₃), 3.79 (dd, 1H, H-6a), 3.61 (dd, 1H, H-6b), 3.49-3.30 (m, 4H, H-2, H-3, H-4, H-5), 1.36 (t, 3H, O-CH₂-CH₃).

### Beispiel 2

52.6 g (128 mmol) 2,3,4,6-Tetra-O-acetyl-α-D-glucopyranosylbromid (α-Acetobromglucose), 41.3 g (128 mmol) Tetrabutylammoniumbromid und 48.4 g (384 mmol) 3-Hydroxy-2-methyl-4-pyranon (Maltol) werden in 450 ml Dichlormethan gelöst und auf 35°C erwärmt. Es werden 450 ml einer 1 molaren NaOH-Lösung zugegeben, und es wird drei Stunden lang bei 35°C heftig gerührt. Anschließend werden 3 l Ethylacetat zugegeben. Die organische Phase wird abgetrennt und es wird nacheinander dreimal mit je 2 l einer 1 molaren NaOH-Lösung, zweimal mit je 2 l Wasser und einmal mit 2 l gesättigter Natriumchlorid-Lösung gewaschen. Die wäßrigen Phasen werden jedesmal mit 200 ml Ethylacetat ausgeschüttelt. Die organische Phase wird über Magnesiumsulfat getrocknet und das Lösungsmittel wird am Rotationsverdampfer entfernt. Zur Reinigung wird in heißem Ethanol gelöst und das Produkt durch Zugabe von Petrolether in der Kälte ausgefällt. Ausbeute: 17.90 g (31%), Schmp.: 147 °C.
¹H-NMR (CDCl₃, Bruker AMX-400): 7.60 (d, 1H, H-6'), 6.30 (d, 1H, H-5'), 5.31 (d, J=7.6, 1H, H-1 ), 5.26 (m, 1H, H-3), 5.16 (dd, 1H, H-2), 5.09 (m, 1H, H-4), 4.17 (dd, 1H, H-6a), 4.10 (dd, 1H, H-6b), 3.63 (ddd, 1H, H-5), 2.28 (s, 3H, 2'-CH₃), 2.11, 2.01, 2.00, 1.99 (je s, je 3H, OCOCH₃).

Zur Deacetylierung werden 1.70 g (3.72 mmol) Maltol-tetra-O-acetyl-β-D-glucopyranosid in 30 ml abs. Methanol gelöst und mit 13 ml einer 1%-igen NaOMe-Lösung versetzt. Nach einer Stunde wird mit Amberlite IR-120 (H⁺) neutralisiert und das Lösungsmittel wird entfernt. Nach säulenchromatographischer Reinigung (50 g Kieselgel, Laufmittel Ethylacetat/Methanol 2:1) erhält man 746 mg (70 %) Maltolglucosid. Schmp.: 109 °C.
¹H-NMR (D₂0, Bruker AMX-400): 7.87 (d, 1H, H-6'), 6.36 (d, 1H, H-5'), 4.72 (dd, J=5.6, 1H, H-1), 3.66 (dd, 1H, H-6a), 3.55 (dd, 1H, H-6b), 3.38-3.34 (m, 2H, H-2, H-3), 3.29 (m, 1H, H-4), 3.21 (m, 1H, H-5), 2.28 (s, 3H, 2'-CH₃).

## Patentansprüche

1. Verfahren zur Herstellung von Glycosiden, bei dem man
a) ein O-geschütztes Halogenid eines Einfach- oder Mehrfachzuckers (I) und einen ungesättigten Alkohol (II), der das Strukturelement 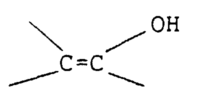 enthält,
b) in einem zweiphasigen Lösungsmittelgemisch und
c) in Gegenwart eines Phasentransferkatalysators zur Reaktion bringt
d) und gegebenenfalls das erhaltene geschützte Glycosid entschützt.

2. Verfahren nach Anspruch 1, dadurch gekennzeichnet, daß man als O-geschütztes Halogenid eines Einfachzuckers (I) α-Acetobromglucose einsetzt.

3. Verfahren nach den Ansprüchen 1 oder 2, dadurch gekennzeichnet, daß man als ungesättigten Alkohol (II) ein Phenol der allgemeinen Formel 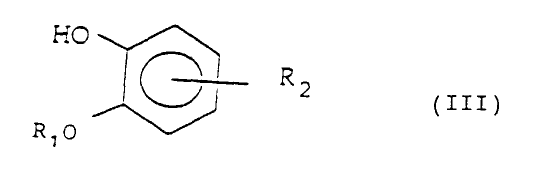 einsetzt, wobei R₁ = Methyl, Ethyl, Propyl, i-Propyl, t-Butyl, Phenyl oder Benzyl und R₂ = -CHO, -CO-CH₃, -CO-C₂H₅, -CO-CH=CH₂, -CO-CH₂-CH=CH₂, -CO-CH=CH-CO₂H, -CO-CH=CH-CH₂OH, -CO-Phenyl oder -CO-Benzyl.

4. Verfahren nach Anspruch 3, dadurch gekennzeichnet, daß man als Phenol (III) Vanillin oder Ethylvanillin einsetzt.

5. Verfahren nach den Ansprüchen 1 oder 2, dadurch gekennzeichnet, daß man als ungesättigten Alkohol (II) einen ungesättigten Alkohol einsetzt, der das Strukturelement 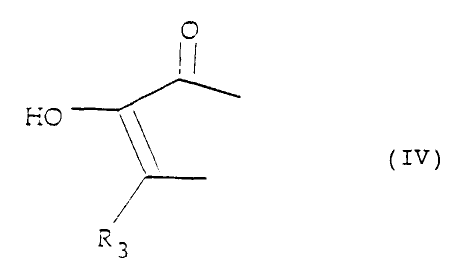 enthält, wobei R₃ = -H, Methyl, Ethyl, Propyl, i-Propyl, t-Butyl, Phenyl oder Benzyl.

6. Verfahren nach Anspruch 5, dadurch gekennzeichnet, daß man als ungesättigten Alkohol (II) Maltol einsetzt.

7. Verfahren nach einem der Ansprüche 1 bis 6, dadurch gekennzeichnet, daß man die Reaktion von Halogenid und Alkohol in einem Gemisch aus einer wäßrigen Natriumhydroxid-Lösung und Methylenchlorid durchführt.

8. Verfahren nach einem der Ansprüche 1 bis 7, dadurch gekennzeichnet, daß man als Phasentransferkatalysator ein quartäres Ammoniumsalz einsetzt.

9. Verfahren nach Anspruch 8, dadurch gekennzeichnet, daß man als quartäres Ammoniumsalz Tetrabutylammoniumbromid einsetzt.

10. Verfahren nach einem der Ansprüche 1 bis 9, dadurch gekennzeichnet, daß man die Reaktion von Halogenid und Alkohol bei einer Temperatur von -10 °C bis 100 °C durchführt.

11. Verfahren nach Anspruch 10, dadurch gekennzeichnet, daß man die Reaktion bei Raumtemperatur durchführt.
